# FASCICULE DE BREVET EUROPEEN

(11) **EP 3 030 138 B1**
(45) Date de publication et mention de la délivrance du brevet: **31.05.2023**
(21) Numéro de dépôt: 14790178.9
(22) Date de dépôt: 05.08.2014
(51) Int. Cl.: A61B 5/0205, A61B 5/03, A61B 5/145, A61B 5/00, G16H 50/30

(54) **SOLUTION INTÉGRÉE DE SUIVI ET DE SURVEILLANCE EN TEMPS RÉEL DE L'ÉTAT PATHOLOGIQUE D'UN PATIENT CÉRÉBRO-LÉSÉ**
INTEGRIERTE LÖSUNG ZUR VERFOLGUNG UND ÜBERWACHUNG DES PATHOLOGISCHEN ZUSTANDES EINES HIRNGESCHÄDIGTEN PATIENTEN IN ECHTZEIT
INTEGRATED SOLUTION FOR TRACKING AND MONITORING, IN REAL TIME, THE PATHOLOGICAL STATE OF A BRAIN-DAMAGED PATIENT

(30) Priorité: 07.08.2013 FR 1357846
(43) Date de publication de la demande: 15.06.2016
(73) Titulaire: Assistance Publique - Hôpitaux de Paris, 75004 Paris (FR)
(72) Inventeur: MATEO, Joaquim, 75018 Paris (FR); VALLEE, Fabrice, 93260 Les Lilas (FR)
(74) Mandataire: IP Trust
(86) Numéro de dépôt international: PCT/FR2014/052031
(87) Numéro de publication internationale: WO 2015/019016

(56) Documents cités:
- US-A1- 2010 010 322
- US-A1- 2010 286 586
- US-A1- 2011 282 169
- CHAMBERLAIN ET AL: "The critical care nurse's role in preventing secondary brain injury in severe head trauma: achieving the balance", 19981201, vol. 11, no. 4, 1 décembre 1998 (1998-12-01), pages 123-129, XP022515138,

## Description

### Domaine de l'invention

La présente invention concerne le domaine du suivi en temps réel des paramètres physiologiques d'un patient cérébro-lésé, particulièrement dans les services de réanimation ou d'urgences.

Les lésions cérébrales se caractérisent par des atteintes des tissus constituant le cerveau. Classiquement, deux types de lésions cérébrales sont distingués :
- les lésions primaires sont dues à une première lésion physique liée à un impact ou à un traumatisme, par exemple lors d'une chute ou d'un accident de la route
- les lésions secondaires peuvent survenir tardivement après une lésion primaire et peuvent se manifester sous la forme d'ischémies cérébrales secondaires. Ces dernières entraînent des lésions dans des zones initialement non atteintes, par conséquent elles aggravent l'état du patient. Ces lésions secondaires sont difficilement prévisibles et rendent la prise en charge des patients cérébro-lésés délicate.

Les patients cérébro-lésés présentent la spécificité de nécessiter une surveillance accrue ainsi qu'une grande vigilance du personnel soignant. En effet, chez les patients cérébro-lésés, les paramètres systémiques ainsi que les paramètres de circulation et d'oxygénation cérébrale évoluent très rapidement, parfois même de l'ordre de la fraction de seconde. Or ces évolutions peuvent avoir une incidence considérable sur l'état global du patient, comme une rapide détérioration ou amélioration. La réactivité du personnel soignant à ces évolutions est primordiale pour prévenir, détecter et traiter les épisodes d'ischémies cérébrales secondaires et par conséquent limiter la survenue de lésions secondaires pouvant être fatales, c'est-à-dire entraîner la mort cérébrale du patient ou pouvant handicaper considérablement le patient par la suite.

### Etat de la technique

Il est connu de l'état de la technique des systèmes de suivi de l'activité cérébrale de patients cérébro-lésés dans les unités de soins intensifs hospitaliers. Ces systèmes peuvent comprendre des moyens de suivis des paramètres systémiques de ces mêmes patients comme la température, la pression artérielle, le rythme cardiaque, la ventilation...

Il est également connu de l'art antérieur des scores de gravité évolutifs qui sont utilisés en réanimation pour grader et classer les malades. Ces scores sont déterminés ponctuellement chez les patients, notamment à leur arrivée en service de réanimation. Ils permettent d'apprécier la gravité du stade pathologique du patient. Parmi ces scores, le score de Glasgow est couramment utilisé à l'arrivée du patient dans un service de réanimation. Il indique l'état de conscience du patient afin d'indiquer au praticien la stratégie thérapeutique à initier pour maintenir les fonctions vitales du patient. Ce score intègre l'ouverture des yeux par le patient, sa réponse verbale et sa réponse motrice. Plus le score est près de zéro, plus l'état de conscience du patient est mauvais, c'est-à-dire qu'il est dans le coma.

L'état de l'art comprend aussi des systèmes combinant le calcul de ces scores à l'acquisition d'informations de suivi du patient comme la fréquence cardiaque, la pression artérielle...

Le brevet EP1903932 décrit un dispositif de mesure du degré d'acuité instantanée du patient. Le système de suivi d'un patient collecte les paramètres physiologiques à intervalles courts et les données cliniques à des intervalles plus longs. Un générateur de score d'acuité composite génère ou actualise un score d'acuité composite indiquant le bien-être d'un patient basé au moins sur un paramètre physiologique mesuré et une donnée d'intervalle plus long. Un écran affiche les valeurs courantes d'au moins un paramètre physiologique sélectionné mesuré, une donnée d'intervalle plus long et le score d'acuité composite.

Un autre brevet de l'art antérieur, le brevet US6416480 porte sur un système et une méthode pour l'acquisition automatique et par ordinateur du score de coma Glasgow pour quantifier le niveau de conscience après un traumatisme cérébral. Ce système réalise l'évaluation du score de Glasgow de patients en état critique sur une période de base. Basé sur la mesure de la réponse à des stimuli de standards physiologiques et verbaux du patient comme l'électromyogramme, l'électro-oculogramme et des expressions simples, le système produit un score de coma qui correspond un à un au score obtenu par un expert humain. Le dispositif utilisé pour l'évaluation automatisée du degré de conscience du patient comprend un ordinateur avec un programme pour évaluer la conscience du patient, au moins une électrode couplé à l'ordinateur pour recueillir une réponse physique, un haut-parleur couplé à l'ordinateur pour produire un signal audio, un microphone couplé à l'ordinateur configuré pour recueillir la réponse audio du patient, et un stimulateur de douleur couplé à l'ordinateur pour générer un stimulus douloureux au patient. La méthode pour évaluer automatiquement le degré de conscience d'un patient utilisant un ordinateur comprend les étapes de détection de la réponse du patient, l'enregistrement de la réponse dans l'ordinateur, la réponse étant caractérisable en nature, l'analyse de la nature caractérisable de la réponse pour déterminer la nature dans l'ordinateur, la catégorisation de la nature de la réponse dans l'ordinateur et la production par l'ordinateur d'un stimulus dépendant de la catégorisation de la réponse.

On connaît aussi de l'état de l'art des solutions de modélisation concernant d'autres pathologies.

La demande de brevet WO2012/122096 décrit des systèmes, des méthodes et des supports lisibles par ordinateur pour fournir une solution d'aide à la décision pour les professionnels médicaux pour optimiser les soins médicaux à travers le suivi des données et le traitement des informations. Dans un autre mode de réalisation, une méthode implémentée par ordinateur pour modéliser les réponses des patients à un traitement dans un domaine médical spécifique consiste à recevoir des données spécifiques au patient, la détermination d'une pluralité d'états possibles du patient en vertu desquels le patient peut être classé, un état instantané d'un patient en vertu duquel le patient peut être classé et déterminer les probabilités de transition du patient d'un des états à tout autre état possible.

Les demandes de brevet publiées US 2010/010311 A1, US 2010/286586 A1 et US 2011/282169 A1, ainsi que l'article par Chamberlain et al., "The critical care nurse's rôle in preventing secondary brain injury in sévère head trauma: achieving the balance", Australian Critical Care vol. 11, no. 4, pages 123 à 129, publié le 1 décembre 1998, décrivent l'évaluation de l'état d'un patient grâce à plusieurs paramètres.

### Inconvénients de l'art antérieur

Les solutions de l'art antérieur proposent l'acquisition de paramètres physiologiques d'un patient en service de réanimation pour le calcul ponctuel ou à intervalle régulier d'un score qualifiant la gravité du patient.

Dans les services de réanimation, le moindre événement interne ou externe peut modifier profondément le pronostic du patient, particulièrement des patients cérébro-lésés. Par exemple, une modification rapide de la pression artérielle systémique peut avoir une incidence sur la pression intracrânienne en entraînant des variations en quelques millisecondes. De même, un changement de température corporelle a une incidence directe sur les paramètres physiologiques du patient.

Certaines solutions de l'art antérieur combinent des paramètres physiopathologiques du patient et des éléments thérapeutiques comme la médication pour le calcul d'un score. Ceci étant, le score établi est global et ne propose pas de corrélation directe entre les paramètres physiopathologiques du patient et le traitement qu'il reçoit. Les solutions de l'art antérieur présentent des méthodes de suivi de l'état du patient en proposant des diagrammes de suivi sur une certaine durée projetant l'évolution du patient depuis son admission mais pas à un instant t uniquement, donc pas en temps réel. Ces solutions ne s'avèrent pas très lisibles et encore moins intuitives pour le personnel soignant. Le personnel devant rechercher le détail des paramètres qui l'intéressent. Ces solutions s'apparentent plus à du pronostic et donc à de la modélisation du devenir de l'état du patient, qu'à du suivi.

Or la prise en charge des patients cérébro-lésés est complexe du fait de phénomènes pouvant se manifester en quelques fractions de seconde. La réactivité du personnel soignant doit être favorisée et optimisée. Il convient de proposer des solutions intégrées gérant les paramètres physiologiques et les données cliniques essentielles en temps réel au lit du patient sans manipulation subsidiaire.

Le problème technique est donc de fournir une information réellement représentative de l'état d'un patient cérébro-lésé avec une réactivité et un temps de réponse adaptés à l'évolution très rapide d'un tel patient.

### Solution apportée par l'invention

En ce sens, la présente invention propose dans son acceptation la plus générale une solution de suivi et de surveillance en temps réel de l'état pathologique d'un patient cérébro-lésé pour favoriser la réactivité et la prise de décision du personnel soignant même peu qualifié.

La présente invention porte sur un programme d'ordinateur pour la mise en oeuvre d'un procédé de suivi et de surveillance en temps réel de l'état pathologique d'un patient cérébro-lésé comprenant l'acquisition d'une pluralité de paramètres cérébraux et systémiques dudit patient et de calcul périodique d' un score de gravité évolutif représentatif de l'état du patient en fonction desdits paramètres cérébraux et systémiques, pour commander un moyen d'affichage de ladite variable représentative de l'état du patient dans lequel lesdits paramètres cérébraux comprennent la pression intracrânienne et la pression de perfusion cérébrale, et dans lequel les paramètres systémiques comprennent la température du patient, l'osmolarité et la pression partielle artérielle en CO₂, ledit procédé comprenant également une étape d' acquisition d'au moins un paramètre clinique comprenant la sédation, lesdits paramètres systémiques et cliniques caractérisant l'intensité thérapeutique employée, et dans lequel le procédé comporte des étapes d'échantillonnage et de segmentation desdits paramètres cérébraux, systémiques et cliniques en fonction de l'appartenance des valeurs mesurées à des intervalles prédéterminés, le nombre d'intervalles étant inférieur à dix, les intervalles étant déterminés, pour chacun desdits paramètres, pour couvrir l'ensemble des valeurs comprises entre une situation correspondant à l'équilibre physiologique et une situation de gravité extrême, chacun desdits intervalles étant associés à une valeur numérique, ledit programme d'ordinateur comprenant en outre des instructions pour commander l'affichage en plus dudit score évolutif sur un même moniteur ou sur des moniteurs différents d' une représentation graphique bidimensionnelle représentative de l'état pathologique dudit patient comprenant, dans une première partie de l'écran d'affichage, l'affichage d'un ensemble de points (Xᵢ, Yᵢ) où i désigne l'un des paramètres, Y désigne la valeur numérique de l'intervalle et X désigne la position de l'intervalle dans ladite représentation graphique, et, dans une partie complémentaire de l'écran d'affichage, l'affichage d'un point (Xₚ, Yₚ) ou P correspond à la pression de perfusion cérébrale.

L'on entend par osmolarité et pression partielle artérielle en CO₂ la valeur stricte correspondant à ces paramètres systémiques ou un reflet de celle-ci. Ainsi l'osmolarité peut être comprise comme la natrémie, la glycémie, l'urémie ou une combinaison d'au moins deux de ces valeurs. De même, la pression partielle artérielle en CO₂ est comprise comme sa valeur stricte mesurée ou son reflet par la concentration mesurée en CO₂ expiré, le EtCO₂ pour end tidal CO₂.

Les paramètres systémiques et cérébraux sont échantillonnés et segmentés de sorte qu'ils sont répartis en maximum dix intervalles et préférentiellement six intervalles. A chaque intervalle est attribué une valeur numérique préférentiellement comprise entre zéro et cinq où zéro correspond à l'état d'équilibre physiologique du patient et cinq à un état de gravité extrême où le pronostique vital du patient est engagé.

L'on comprend que la représentation graphique comprend deux parties, l'une pour la projection de la valeur numérique de l'intervalle comprenant la mesure de la pression de perfusion cérébrale, l'autre pour la projection des valeurs numériques correspondant aux valeurs mesurées des autres paramètres cérébraux, systémiques et cliniques, notamment la pression intracrânienne, la température du patient, l'osmolarité, la pression partielle artérielle en CO₂ et la sédation.

Avantageusement, l'on calcule une combinaison des valeurs numériques correspondant chacune à l'un des intervalles pour déterminer ledit score de gravité évolutif et pronostic dudit patient.

Le score de gravité évolutif et pronostic dudit patient est calculé en temps réel selon les paramètres systémiques, cérébraux et cliniques acquis. Ce score qualifie le pronostique vital du patient.

L'on comprend que le score de gravité évolutif et pronostic dudit patient peut également être établi par la liaison sur la représentation graphique des différentes valeurs numériques projetées.

Un autre aspect de l'invention porte sur un programme d'ordinateur pour la mise en oeuvre du procédé. Ce programme d'ordinateur comprend des moyens d'échantillonnage et de segmentation desdits paramètres cérébraux, systémiques et cliniques d'un patient et des moyens de calcul d'un score de gravité évolutif et pronostique à partir des valeurs numériques correspondant à l'intervalle où se positionne les paramètres dudit patient, ledit score de gravité évolutif et pronostique étant caractéristique de l'état pathologique du patient.

Avantageusement, l'étape de segmentation consiste à attribuer à chacun desdits paramètres une valeur numérique comprise entre 0 et 9 et de préférence entre 0 et 5 au paramètres en fonction de l'intervalle dans lequel la valeur dudit paramètre est incluse.

Avantageusement, le programme d'ordinateur pour la mise en oeuvre du procédé commande la visualisation, à partir desdites valeurs numériques d'intervalle des paramètres cérébraux, systémiques et cliniques, d'une représentation graphique bidimensionnelle représentative de l'état pathologique dudit patient.

Avantageusement, le programme d'ordinateur pour la mise en oeuvre du procédé comporte des moyens d'affichage dudit score de gravité évolutif et pronostique du patient et de ladite représentation graphique bidimensionnelle caractéristiques de l'état pathologique du patient sur un équipement.

L'on comprend que les scores de gravité évolutifs et pronostics et les représentations graphiques sont stockés et archivés, le programme d'ordinateur permettant d'aller rechercher à n'importe quel moment ces mêmes données pour établir un profil de l'évolution du patient depuis son admission dans le service de réanimation jusqu'à une date et un horaire désirés.

Selon une variante, le programme d'ordinateur pour la mise en oeuvre du procédé comporte des moyens d'entrées-sorties aptes à dialoguer avec un équipement d'acquisition des paramètres cérébraux, systémiques et cliniques.

Un autre aspect de l'invention porte sur un équipement commandé par le programme d'ordinateur selon l'invention pour le suivi en temps réel de l'état pathologique d'un patient cérébro-lésé. Cet équipement comporte un calculateur commandé par ledit programme d'ordinateur, ledit équipement comportant des moyens de restitution en continu et en temps réel de ladite représentation graphique bidimensionnelle et dudit score de gravité évolutif et pronostique caractéristiques de l'état pathologique du patient.

Avantageusement, l'équipement commandé par un tel programme pour le suivi en temps réel de l'état pathologique d'un patient cérébro-lésé comporte au moins un moniteur pour l'affichage en temps réel dudit score de gravité évolutif et pronostique et de ladite représentation graphique bidimensionnelle caractéristiques de l'état pathologique du patient.

L'on comprend que le score et la représentation graphique peuvent être affichés sur un même moniteur ou sur des moniteurs différents.

Un autre aspect de l'invention porte sur une plateforme de suivi et de surveillance en temps réel de l'état pathologique d'un patient cérébro-lésé comprenant une pluralité d'appareillages de mesure et d'acquisition en temps réel de paramètres physiologiques d'un patient, ladite plateforme comprend l' équipement d'acquisition desdits paramètres physiologiques et cliniques selon l'invention et des moyens de restitution en continu et en temps réel dudit score de gravité évolutif et pronostique d'un patient ainsi que d' établissement d'une représentation graphique bidimensionnelle caractéristiques de l'état pathologique d'un patient, ledit équipement étant commandé par ledit programme d'ordinateur pour la détermination dudit score de gravité évolutif et pronostique d'un patient ainsi que de ladite représentation graphique bidimensionnelle caractéristiques de l'état pathologique d'un patient .

Avantageusement, la plateforme de suivi et de surveillance en temps réel de l'état pathologique d'un patient cérébro-lésé comprend un calculateur de statistiques de suivi de l'état du patient et des moyens d'affichage desdits statistiques sur au moins un moniteur.

Selon une variante, la plateforme de suivi et de surveillance en temps réel de l'état pathologique d'un patient cérébro-lésé est multimodale.

Selon une autre variante, les appareillages sont synchronisés.

### Description

La présente invention sera mieux comprise à la lecture de la description suivante.

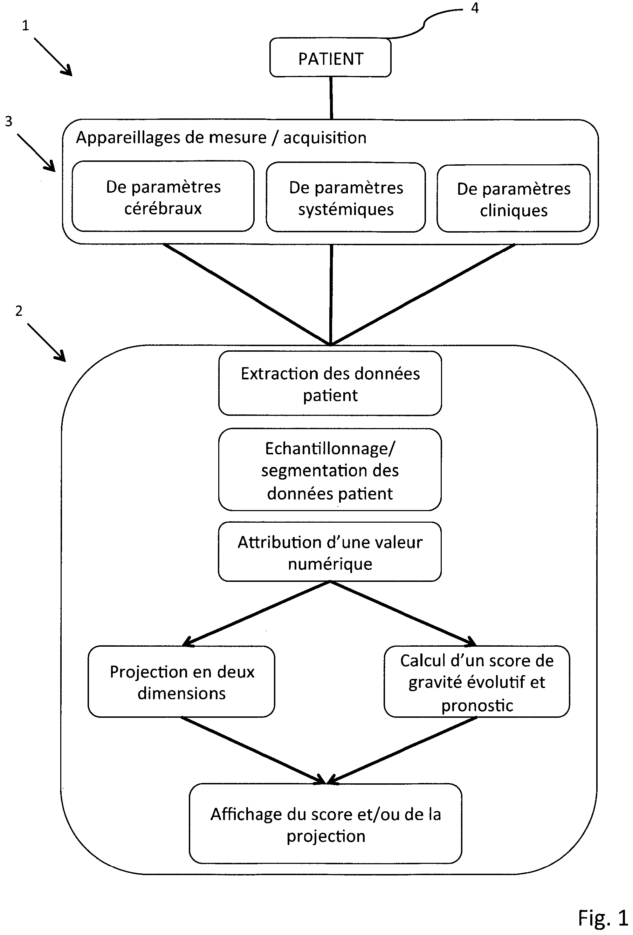

La figure 1 est une représentation schématique de la solution intégrée de suivi et de surveillance en temps réel de l'état pathologique d'un patient cérébro-lésé conforme à l'invention.

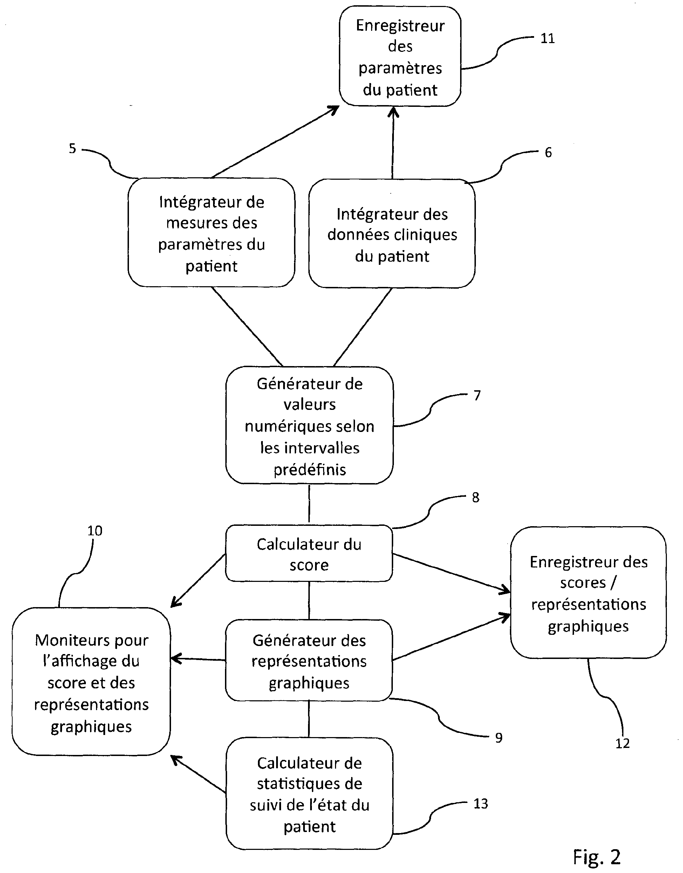

La figure 2 est une représentation schématique de l'équipement conforme à l'invention.

Dans les services de réanimation ou d'urgences, les patients cérébro-lésés font l'objet d'une prise en charge particulière. Ces patients nécessitent une attention accrue du fait de dégradation rapide pouvant survenir à tout instant.

La présente invention propose donc une plateforme (1) de suivi et de surveillance en temps réel d'un patient cérébro-lésé comportant un équipement (2) commandé par un programme d'ordinateur pour la mise en oeuvre d'un procédé de suivi et de surveillance en temps réel de l'état pathologique d'un patient. Une pluralité d'appareillages de mesures (3) de paramètres physiologiques est disposée autour du lit du patient (4). Ainsi sont acquis en temps réel et en continu des paramètres systémiques comme par exemple la pression sanguine, la fréquence cardiaque, la température corporelle ; et des paramètres de circulation et d'oxygénation cérébrale.

De cette pluralité de données, six de ces paramètres sont extraits et intégrés par au moins un intégrateur (5, 6) du programme d'ordinateur pour la mise en oeuvre du procédé conforme à l'invention. Ces paramètres sont de trois sortes :
- des paramètres cérébraux comme la pression de perfusion cérébrale et la pression intracrânienne
- des paramètres systémiques comme la température corporelle, l'osmolarité et la pression partielle artérielle en CO₂
- des paramètres cliniques comme la sédation.

Les paramètres systémiques et cliniques caractérisent l'intensité thérapeutique utilisée chez le patient.

Chaque paramètre mesuré unitairement sur la plateforme par un appareillage est échantillonné et segmenté par un générateur de valeurs numériques (7) en fonction de l'appartenance de la valeur mesurée à des intervalles déterminés. Ces intervalles sont définis pour chaque paramètre. Chaque paramètre est donc segmenté en six intervalles de répartition homogène et représentative d'un stade de gravité de l'état pathologique d'un patient quelconque. A titre d'exemple, la pression de perfusion cérébrale est segmentée selon six intervalles où le premier comprend des valeurs de pression supérieure à 60 mm Hg et le dernier comprend des valeurs de pression inférieures à 40 mm Hg, l'incrémentation entre les intervalles étant de 5 mm Hg. A chaque intervalle correspond une valeur numérique comprise entre 0 et 5. Ainsi la valeur mesurée de pression de perfusion chez le patient sera catégorisée sur l'intervalle comprenant la valeur mesurée. A titre d'exemple, si la pression de perfusion cérébrale mesurée chez le patient est de 51 mm Hg, la valeur numérique de l'intervalle correspondant sera de 2. Le programme d'ordinateur procède de même pour les autres paramètres entrant en jeu dans le procédé.

Dans un deuxième temps, le programme d'ordinateur par un calculateur (8), conformément au procédé, combine les valeurs numériques obtenues pour chaque paramètre et calcule un score de gravité évolutif en temps réel qui est affiché sur au moins un moniteur de la plateforme.

Plus le score est faible, meilleur est le pronostic du patient. A l'inverse, plus le score est élevé, plus l'état du patient est sévère et nécessitera d'augmenter et d'adapter l'intensité thérapeutique utilisée.

Egalement, le programme d'ordinateur par un générateur de représentation graphique (9) conformément au procédé réalise une projection sur une représentation graphique bidimensionnelle des valeurs numériques obtenues pour chaque paramètre qui est affichée sur l'équipement conformément à l'invention par au moins un moniteur (10). Cette représentation graphique comprend deux parties :
- une partie haute pour la projection de la valeur numérique correspondant à l'intervalle où se situe la valeur mesurée de pression de perfusion cérébrale.
- une partie basse dans laquelle les valeurs numériques des autres paramètres sont projetées. Cette partie basse correspond à l'intensité thérapeutique utilisée chez ce patient.

Les différentes valeurs numériques sont reliées entre elles pour dessiner une forme représentative de l'état pathologique du patient. Cette forme confère un profil pronostic au patient.

Par ailleurs, cette représentation graphique ainsi que le score de gravité évolutif et pronostic du patient sont affichés sur un moniteur (10) qui peut être dédié au sein de la plateforme (1) de suivi et de surveillance de l'état pathologique d'un patient cérébro-lésé ou s'additionner sur tout ou partie des moniteurs des appareillages de la plateforme.

La plateforme comprend également des moyens de stockage et d'archivage des données mesurées (11), des représentations graphiques bidimensionnelles et des scores de gravité évolutifs et pronostics (12). Ainsi, des données antérieures peuvent être restituées et affichées à la demande. Egalement, le programme par un calculateur (13) permet l'établissement d'une courbe de suivi du score de gravité évolutif et pronostic du patient ainsi que de la représentation graphique bidimensionnelle, et ce, tout au long de la prise en charge du patient dans les services de soins.

## Revendications

1. - Programme d'ordinateur pour la mise en œuvre d'un procédé de suivi et de surveillance en temps réel de l'état pathologique d'un patient (4) cérébro-lésé comprenant l'obtention de données de plusieurs appareils de mesure adaptés pour l'acquisition d'une pluralité de paramètres cérébraux et systémiques dudit patient (4) et de calcul périodique d'un score de gravité évolutif représentatif de l'état du patient (4) en fonction desdits paramètres cérébraux et systémiques, pour commander un moyen d'affichage dudit score de gravité évolutif représentatif de l'état du patient (4) affiché sur au moins un moniteur et dans lequel lesdits paramètres cérébraux comprennent la pression intracrânienne et la pression de perfusion cérébrale, et dans lequel les paramètres systémiques comprennent la température du patient, l'osmolarité et la pression partielle artérielle en CO₂, et ledit procédé comprenant également une étape d' acquisition d'au moins un paramètre clinique comprenant la sédation, lesdits paramètres systémiques et cliniques caractérisant l'intensité thérapeutique employée et dans lequel le procédé comporte des étapes d'échantillonnage et de segmentation desdits paramètres cérébraux, systémiques et cliniques en fonction de l'appartenance des valeurs mesurées à des intervalles prédéterminés, le nombre d'intervalles étant inférieur à dix, les intervalles étant déterminés, pour chacun desdits paramètres, pour couvrir l'ensemble des valeurs comprises entre une situation correspondant à l'équilibre physiologique et une situation de gravité extrême, chacun desdits intervalles étant associés à une valeur numérique, ledit programme d'ordinateur comprenant en outre des instructions pour commander l'affichage en plus dudit score évolutif sur un même moniteur ou sur des moniteurs différents d'une représentation graphique bidimensionnelle représentative de l'état pathologique dudit patient comprenant, dans une première partie de l'écran d'affichage, l'affichage d'un ensemble de points (Xᵢ, Yᵢ) où i désigne l'un des paramètres, Y désigne la valeur numérique de l'intervalle et X désigne la position de l'intervalle dans ladite représentation graphique, et, dans une partie complémentaire de l'écran d'affichage, l'affichage d'un point (Xₚ, Yₚ) ou P correspond à la pression de perfusion cérébrale

2. - Programme d'ordinateur selon la revendication 1 **caractérisé en ce que** l'étape de segmentation dudit procédé de suivi et de surveillance consiste à attribuer à chacun desdits paramètres une valeur numérique comprise entre 0 et 9 et de préférence entre 0 et 5 aux paramètres en fonction de l'intervalle dans lequel la valeur dudit paramètre est incluse.

3. - Programme d'ordinateur selon la revendication 1 **caractérisé en ce que** l'on calcule une combinaison des valeurs numériques correspondant chacune à l'un des intervalles pour déterminer un score de gravité évolutif et pronostic dudit patient.

4. - Equipement d'acquisition et d'enregistrement de paramètres physiologiques (2) pour la mise en oeuvre du programme d'ordinateur selon l'une quelconque des revendications 1 à 3, pour le suivi en temps réel de l'état pathologique d'un patient cérébro-lésé ledit équipement comportant au moins un calculateur (8, 13) commandé par ledit programme d'ordinateur conforme à l'une des revendications 1 à 3, ledit équipement comportant des moyens de restitution en continu et en temps réel de ladite représentation graphique bidimensionnelle et dudit score de gravité évolutif et pronostique caractéristiques de l'état pathologique du patient.

5. - Equipement selon la revendication 4 **caractérisé en ce qu'**il comporte au moins un moniteur (10) pour l'affichage en temps réel dudit score de gravité évolutif et pronostique et de ladite représentation graphique bidimensionnelle caractéristiques de l'état pathologique du patient.

6. - Equipement selon la revendication 4 ou 5 **caractérisé en ce qu'**il comporte des moyens d'entrées-sorties aptes à dialoguer avec un équipement d'acquisition des paramètres cérébraux, systémiques et cliniques (11).

7. - Plateforme (1) pour la mise en oeuvre d'un programme d'ordinateur selon l'une quelconque des revendications 1 à 3 comprenant une pluralité d'appareillages (3) de mesure et d'acquisition en temps réel de paramètres physiologiques d'un patient ladite plateforme comprenant un équipement (2) d'acquisition et d'enregistrement desdits paramètres physiologiques et cliniques conforme aux revendications 4 à 6, et des moyens de restitution en continu et en temps réel dudit score de gravité évolutif et pronostique d'un patient ainsi que d'établissement de ladite représentation graphique bidimensionnelle caractéristiques de l'état pathologique d'un patient, pour la détermination dudit score de gravité évolutif et pronostique d'un patient ainsi que de ladite représentation graphique bidimensionnelle caractéristiques de l'état pathologique d'un patient.

8. - Plateforme selon la revendication 7 **caractérisé en ce qu'**elle comprend un calculateur (13) de statistiques de suivi de l'état du patient et des moyens d'affichage desdits statistiques sur au moins un moniteur (10).

9. - Plateforme selon l'une des revendications 7 et 8 **caractérisé en ce qu'**elle est multimodale.

10. - Plateforme selon l'une des revendications 7 à 9 **caractérisé en ce que** les appareillages sont synchronisés.

## Patentansprüche

1. Computerprogramm für die Ausführung eines Verfahrens zum Verfolgen und Überwachen des pathologischen Zustands eines hirngeschädigten Patienten (4) in Echtzeit, umfassend das Erhalten von Daten von mehreren Messgeräten, die zum Erfassen einer Vielzahl von zerebralen und systemischen Parametern des Patienten (4) angepasst sind, und zum periodischen Berechnen einer evolutiven Schweregradbewertung, die den Zustand des Patienten (4) darstellt, in Abhängigkeit von den zerebralen und systemischen Parametern, zum Steuern eines Mittels zum Anzeigen der evolutiven Schweregradbewertung, die den Zustand des Patienten (4) darstellt und auf mindestens einem Monitor angezeigt wird, und wobei die zerebralen Parameter den intrakraniellen Druck und den zerebralen Perfusionsdruck umfassen, und wobei die systemischen Parameter die Temperatur des Patienten, die Osmolarität und den arteriellen CO₂-Partialdruck umfassen, und das Verfahren auch umfassend einen Schritt des Erfassens mindestens eines klinischen Parameters, der die Sedierung umfasst, wobei die systemischen und klinischen Parameter die angewandte therapeutische Intensität charakterisieren, und wobei das Verfahren Schritte einer Probenahme und Segmentierung der zerebralen, systemischen und klinischen Parameter in Abhängigkeit von der Zugehörigkeit der gemessenen Werte zu vorbestimmten Intervallen aufweist, wobei die Anzahl Intervalle weniger als zehn beträgt, wobei die Intervalle für jeden der Parameter so bestimmt werden, dass sie den Satz der Werte zwischen einer Situation, die dem physiologischen Gleichgewicht entspricht, und einer Situation mit extremem Schweregrad abdecken, wobei jedes der Intervalle mit einem numerischen Wert verknüpft ist, das Computerprogramm außerdem umfassend Anweisungen zum Steuern der Anzeige zusätzlich zu der evolutiven Schweregradbewertung auf demselben Monitor oder auf verschiedenen Monitoren einer zweidimensionalen grafischen Darstellung, die den Krankheitszustand des Patienten darstellt, umfassend in einem ersten Teil des Anzeigebildschirms die Anzeige eines Satzes von Punkten (Xi, Yᵢ), wobei i einen der Parameter bezeichnet, Y den numerischen Wert des Intervalls bezeichnet und X die Position des Intervalls in der grafischen Darstellung bezeichnet, und in einem komplementären Teil des Anzeigebildschirms die Anzeige eines Punkts (Xₚ, Yₚ), wobei P dem zerebralen Perfusionsdruck entspricht.

2. Computerprogramm nach Anspruch 1, **dadurch gekennzeichnet, dass** der Segmentierungsschritt des Verfahrens zum Verfolgen und Überwachen darin besteht, jedem der Parameter einen numerischen Wert zwischen 0 und 9 und vorzugsweise zwischen 0 und 5 den Parametern in Abhängigkeit von dem Intervall zuzuordnen, in dem der Wert des Parameters enthalten ist.

3. Computerprogramm nach Anspruch 1, **dadurch gekennzeichnet, dass** eine Kombination der numerischen Werte berechnet wird, die jeweils einem der Intervalle entsprechen, um eine evolutive und prognostische Schweregradbewertung des Patienten zu bestimmen.

4. Ausrüstung zum Erfassen und Aufzeichnen physiologischer Parameter (2) für die Ausführung des Computerprogramms nach einem der Ansprüche 1 bis 3 zum Verfolgen des pathologischen Zustands eines hirngeschädigten Patienten in Echtzeit, wobei die Ausrüstung mindestens einen Rechner (8, 13) aufweist, der durch das Computerprogramm nach einem der Ansprüche 1 bis 3 gesteuert wird, wobei die Ausrüstung Mittel für eine kontinuierliche und in Echtzeit erfolgende Wiedergabe der zweidimensionalen grafischen Darstellung und der evolutiven und prognostischen Schweregradbewertung aufweist, die für den pathologischen Zustand des Patienten charakteristisch sind.

5. Ausrüstung nach Anspruch 4, **dadurch gekennzeichnet, dass** sie mindestens einen Monitor (10) für eine Echtzeitanzeige der evolutiven und prognostischen Schweregradbewertung und der zweidimensionalen grafischen Darstellung aufweist, die für den pathologischen Zustand des Patienten charakteristisch sind.

6. Ausrüstung nach Anspruch 4 oder 5, **dadurch gekennzeichnet, dass** sie Mittel für eine Eingabe/Ausgabe aufweist, die geeignet sind, mit einer Ausrüstung zum Erfassen der zerebralen, systemischen und klinischen Parameter (11) zu kommunizieren.

7. Plattform (1) für die Ausführung eines Computerprogramms nach einem der Ansprüche 1 bis 3, umfassend eine Vielzahl von Geräten (3) zum Messen und Erfassen physiologischer Parameter eines Patienten in Echtzeit, die Plattform umfassend eine Ausrüstung (2) zum Erfassen und Aufzeichnen der physiologischen und klinischen Parameter nach den Ansprüchen 4 bis 6, und Mittel für die kontinuierliche und in Echtzeit erfolgende Wiedergabe der evolutiven und prognostischen Schweregradbewertung eines Patienten sowie zum Erstellen der zweidimensionalen grafischen Darstellung, die für den pathologischen Zustand eines Patienten charakteristisch ist, für die Bestimmung der evolutiven und prognostischen Schweregradbewertung eines Patienten sowie für die zweidimensionale grafische Darstellung, die für den pathologischen Zustand eines Patienten charakteristisch sind.

8. Plattform nach Anspruch 7, **dadurch gekennzeichnet, dass** sie einen Rechner (13) für Statistiken zum Verfolgen des Zustands des Patienten und Mittel zum Anzeigen der Statistiken auf mindestens einem Monitor (10) umfasst.

9. Plattform nach einem der Ansprüche 7 und 8,
**dadurch gekennzeichnet, dass** sie multimodal ist.

10. Plattform nach einem der Ansprüche 7 bis 9,
**dadurch gekennzeichnet, dass** die Geräte synchronisiert sind.

## Claims

1. Computer program for implementing a method for tracking and monitoring, in real time, the pathological state of a brain-damaged patient (4), comprising obtaining data of a plurality of measurement devices suitable for acquiring a plurality of cerebral and systemic parameters of said patient (4) and for periodically calculating a scalable severity score, which is representative of the state of the patient (4), on the basis of said cerebral and systemic parameters, for controlling a means for displaying said scalable severity score which is representative of the state of the patient (4) and displayed on at least one monitor and wherein said cerebral parameters comprise intracranial pressure and cerebral perfusion pressure, and wherein the systemic parameters comprise the temperature of the patient, osmolarity and CO₂ arterial partial pressure, and said method also comprising a step of acquiring at least one clinical parameter comprising sedation, said systemic and clinical parameters characterizing the therapeutic intensity used and wherein the method comprises steps of sampling and segmenting said cerebral, systemic and clinical parameters on the basis of the measured values belonging to predetermined intervals, the number of intervals being less than ten, the intervals being determined, for each of said parameters, to cover all of the values between a situation corresponding to physiological equilibrium and an extremely serious situation, each of said intervals being associated with a numerical value, said computer program further comprising instructions for controlling the display in addition to said scalable score on the same monitor or on different monitors of a two-dimensional graphical representation which is representative of the pathological state of said patient, comprising, in a first portion of the display screen, the display of a set of points (Xi, Yi), where i denotes one of the parameters, Y denotes the numerical value of the interval and X denotes the position of the interval in said graphical representation, and, in a complementary portion of the display screen, the display of a point (Xₚ, Yₚ), where P corresponds to cerebral perfusion pressure.

2. Computer program according to claim 1, **characterized in that** the step of segmenting said tracking and monitoring method consists in assigning a numerical value between 0 and 9 to each of said parameters and preferably between 0 and 5 to the parameters on the basis of the interval in which the value of said parameter is included.

3. Computer program according to claim 1, **characterized in that** a combination of the numerical values which each correspond to one of the intervals is calculated in order to determine a scalable and prognostic severity score of said patient.

4. Apparatus for acquiring and recording physiological parameters (2) for implementing the computer program according to any of claims 1 to 3, for tracking, in real time, the pathological state of a brain-damaged patient, said apparatus comprising at least one calculator (8, 13) controlled by said computer program according to any of claims 1 to 3, said apparatus comprising means for reproducing, continuously and in real-time, said two-dimensional graphical representation and said scalable and prognostic severity score, which are characteristic of the pathological state of the patient.

5. Apparatus according to claim 4, **characterized in that** it comprises at least one monitor (10) for displaying, in real time, said scalable and prognostic severity score and said two-dimensional graphical representation, which are characteristic of the pathological state of the patient.

6. Apparatus according to claim 4 or 5, **characterized in that** it comprises input-output means which are capable of communicating with an apparatus for acquiring cerebral, systemic and clinical parameters (11).

7. Platform (1) for implementing a computer program according to any of claims 1 to 3, comprising a plurality of devices (3) for measuring and acquiring, in real time, physiological parameters of a patient, said platform comprising an apparatus (2) for acquiring and recording said physiological and clinical parameters according to claims 4 to 6, and means for reproducing, continuously and in real time, said scalable and prognostic severity score of a patient as well as establishing said two-dimensional graphical representation, which are characteristic of the pathological state of a patient, for determining said scalable and prognostic severity score of a patient as well as said two-dimensional graphical representation, which are characteristic of the pathological state of a patient.

8. Platform according to claim 7, **characterized in that** it comprises a statistics calculator (13) for tracking the state of the patient and means for displaying said statistics on at least one monitor (10).

9. Platform according to any of claims 7 and 8, **characterized in that** it is multimodal.

10. Platform according to any of claims 7 to 9, **characterized in that** the devices are synchronized.
